# EUROPEAN PATENT APPLICATION

(11) **EP 4 512 802 A1**
(43) Date of publication of application: **26.02.2025**
(21) Application number: 23791119.3
(22) Date of filing: 13.04.2023
(51) Int. Cl.: C07D 279/20, C07D 279/18

(54) **PURIFICATION METHOD FOR ELECTRON MEDIATOR**

(30) Priority: 19.04.2022 CN 202210407326; 19.04.2022 CN 202210407840; 24.06.2022 CN 202210730623
(71) Applicant: Leadway (HK) Limited, Sheung Wan, Hong Kong (CN)
(72) Inventor: LU, Xiaofeng, Hangzhou, Zhejiang 310030 (CN); XU, Junjie, Hangzhou, Zhejiang 310030 (CN)
(74) Representative: Schiweck Weinzierl Koch Patentanwälte Partnerschaft mbB
(86) International application number: PCT/CN2023/088087
(87) International publication number: WO 2023/202464

(57) **Abstract**

A purification method for an electron mediator on a biosensor, the electron mediator having an amino. The purification method comprises the following steps: weighing an electron mediator with low purity; reacting an amino protection reagent with the electron mediator to obtain an amino-protected electron mediator, dissolving the amino-protected electron mediator in an organic solvent, and carrying out extraction to obtain an amino-protected electron mediator with high purity; and reacting the amino-protected electron mediator with high purity with a deprotection reagent for amino deprotection, thus obtaining an electron mediator with high purity. The method for purification achieves a very large treatment capacity in one time, effectively improves the purification efficiency of the electron mediator, and reduces the preparation cost. The electron mediator with high purity obtained from purification, serving as one of the components in the reagent on a biosensor, can be used for preparation of biosensors such as a blood glucose test biosensor.

## Description

### Field of the Invention

The present invention relates to a method for purification of an electron mediator, and in particular to a method for purification of an electron mediator used in test reagents such as a blood glucose test reagent.

### Background of the Invention

Diabetes is a disease caused by secretion defects or reduced biological effects of insulin. At present, there has been no medical method that can radically cure diabetes. Active early screening and corresponding prevention and treatment are effective means to control the rise of blood glucose. It is a common method at present to use enzyme electrode based blood glucose meters for daily monitoring of blood glucose levels.

In an enzyme electrode based blood glucose biosensor (also referred to as a blood glucose test strip), the enzyme adopted is a macromolecule, the redox active center of which is encapsulated in an enzyme protein molecule. It is difficult to carry out electron transfer between the center and the electrode surface, and even if direct electron transfer can take place, the electron transfer rate thereof is also relatively slow. Therefore, an electron mediator will be added to the enzyme electrode based test strip to form an electron mediator based biosensor.

Azure C, which is a biological stain, as well as a redox indicator and an electron mediator, is widely used in biochemical and electrochemical research. For example, Azure C is used as an electron mediator for blood glucose biosensors. Commercially available Azure C has a relatively low purity, basically between 40% and 50%. It also contains a series of structurally similar substances such as Azure A, Azure B, and thionine. The proportion of each component in Azure C produced by different manufacturers varies greatly, and even if they are from the same manufacturer, there are also significant lot-to-lot variations, leading to significant uncertainties in practical applications of commercially available Azure C. In order to obtain more precise dosage of Azure C and improve the using effect, it is urgent to obtain Azure C with high purity. Commercially available Azure C exists in the form of hydrochloride, but its solubility in water or organic solvents is very poor, and as the polarities of other components are very approximate to that of Azure C, it is very difficult to purify Azure C. In view of the chemical properties of Azure C, HPLC preparation may be selected for purification to obtain Azure C with high purity, the purity of which is greater than or equal to 99%. However, due to the solubility issue of Azure C and the low sample loading quantity required for HPLC preparation, the sample loading quantity in one time is generally about 5mg. 1-2mg of Azure C can be obtained through a single purification, resulting in low purification efficiency of HPLC preparation and very high purification costs.

### Summary of the Invention

In order to solve the problems in the prior art, the present invention protects an amino on an electron mediator through an amino protection reagent to obtain an intermediate prone to separation, and removes the protecting group on the amino through deprotection to obtain an electron mediator with high purity.

The present invention provides a method for purification of an electron mediator, the electron mediator having an amino, wherein the method comprises the following steps: weighing an electron mediator with low purity; reacting an amino protection reagent with the electron mediator to obtain an amino-protected electron mediator, dissolving the amino-protected electron mediator in an organic solvent, and carrying out extraction to obtain an amino-protected electron mediator with high purity; and reacting the amino-protected electron mediator with high purity with a deprotection reagent for amino deprotection, thus obtaining an electron mediator with high purity.

Further, the electron mediator is selected from Azure C.

Further, the amino protection reagent is selected from fluorenylmethyloxycarbonyl chloride (Fmoc-Cl), di-tert-butyl dicarbonate (Boc2O), triphenylchloromethane (Trt-Cl), carbobenzoxy chloride (Cbz-Cl), paratoluensulfonyl chloride (Tos-Cl) or trifluoroacetic anhydride (TFAA).

Further, the structural formula of the amino-protected electron mediator is 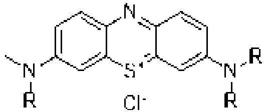 wherein R = H or X, X = amino protecting group, and the number of the amino protecting groups X varies from 1 to 3.

Further, the amino protecting group is, for example, Fmoc, Boc, Trt, Cbz, Tos or TFA.

Further, the deprotection reagent is selected from diethylamine, trifluoroacetic acid, hydrogen bromide/acetic acid, or sodium hydroxide.

Further, the deprotection reagent diethylamine or piperidine is used for deprotection of Fmoc, trifluoroacetic acid is used for deprotection of Boc or Trt, hydrogen bromide/acetic acid is used for deprotection of Cbz or Tos, and sodium hydroxide is used for deprotection of TFA.

Further, the method for extracting an amino-protected electron mediator with high purity is column chromatography.

The electron mediator is exemplified by Azure C. The present invention purifies Azure C by a simple chemical method. At first, the amino protection reagent is used to react with Azure C to obtain amino-protected Azure C, and the amino protection reagent is selected from fluorenylmethyloxycarbonyl chloride (Fmoc-Cl), di-tert-butyl dicarbonate (Boc2O), triphenylchloromethane (Trt-Cl), carbobenzoxy chloride (Cbz-Cl), paratoluensulfonyl chloride (Tos-Cl) or trifluoroacetic anhydride (TFAA). Amino-protected Azure C is prone to dissolving in various organic solvents, and amino-protected Azure C with high purity can be obtained through methods such as column chromatography. Then, amino deprotection can be carried out to obtain Azure 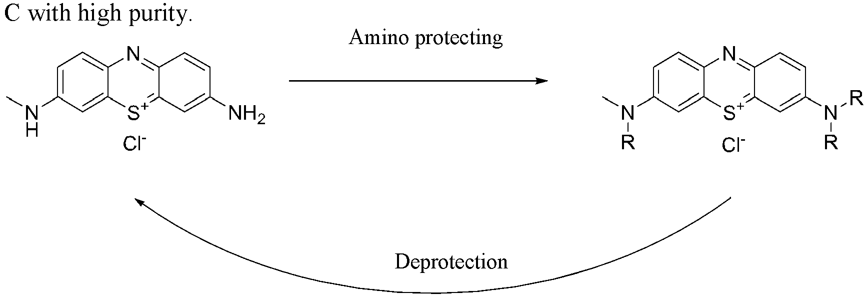 wherein R = H or X, X = amino protecting group (such as Fmoc, Boc, Trt, Cbz, Tos or TFA), different amino protection reagents are utilized, and the number of the amino protecting groups X varies from 1 to 3.

In which:
Azure C: 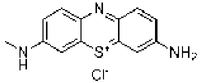
Fmoc-Cl: 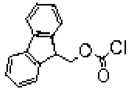
(Fmoc)₂-Azure C: 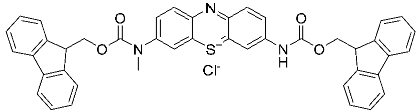
Boc₂O: 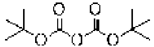
(Boc)₂-Azure C: 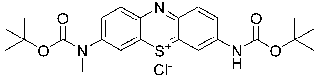
Trt-Cl: 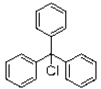
Trt-Azure C:

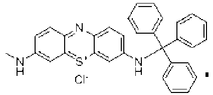

One of the objects of the present invention is to provide a method for purification of an electron mediator with an amino, wherein the method comprises the following steps: weighing an electron mediator with low purity; reacting an amino protection reagent with the electron mediator to obtain an amino-protected electron mediator, dissolving the amino-protected electron mediator in an organic solvent, and carrying out extraction to obtain an amino-protected electron mediator with high purity; and reacting the amino-protected electron mediator with high purity with a deprotection reagent for amino deprotection, obtaining an electron mediator with high purity.

Further, the electron mediator is selected from Azure C.

Further, the amino protection reagent is selected from fluorenylmethyloxycarbonyl chloride (Fmoc-Cl), di-tert-butyl dicarbonate (Boc2O), triphenylchloromethane (Trt-Cl), carbobenzoxy chloride (Cbz-Cl), paratoluensulfonyl chloride (Tos-Cl) or trifluoroacetic anhydride (TFAA).

Further, the structural formula of the amino-protected electron mediator is 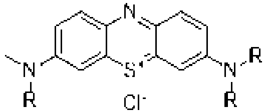 wherein R = H or X, X = amino protecting group, and the number of the amino protecting groups X varies from 1 to 3.

Further, the amino protecting group is selected from Fmoc, Boc, Trt, Cbz, Tos or TFA.

Further, the deprotection reagent is selected from diethylamine, trifluoroacetic acid, hydrogen bromide/acetic acid, or sodium hydroxide.

Further, the deprotection reagent diethylamine or piperidine is used for deprotection of Fmoc, trifluoroacetic acid is used for deprotection of Boc or Trt, hydrogen bromide/acetic acid is used for deprotection of Cbz or Tos, and sodium hydroxide is used for deprotection of TFA.

Further, the method for extracting an amino-protected electron mediator with high purity is column chromatography.

One of the objects of the present invention is to provide a method for purification of Azure C using fluorenylmethyloxycarbonyl chloride (Fmoc-Cl) as an amino protection reagent, and the method comprises the following steps: preparing a mixed solution of Azure C with low purity, sodium carbonate and dioxane; adding Fmoc-Cl to the mixed solution for reaction to obtain a viscous product; dissolving the viscous product in an organic solvent, filtering out insoluble impurities, washing the filtrate with distilled water, and carrying out drying and concentration to obtain an intermediate (Fmoc)₂-Azure C; and dissolving the intermediate (Fmoc)₂-Azure C in DMF, adding diethylamine for reaction, then adding ether to separate out a black solid, and carrying out vacuum filtration, washing and drying to obtain Azure C with high purity. Further, Fmoc-Cl is added to the mixed solution cooled down to 10°C.

Further, Fmoc-Cl is added to react in an ice-water bath at first and then react at room temperature.

Further, the viscous product is dissolved in dichloromethane.

Further, the intermediate (Fmoc)₂-Azure C is dissolved in DMF, and diethylamine is added in an ice-water bath for reaction in the ice-water bath.

Further, sodium carbonate is weighed and stirred in water for dissolving, then dioxane and Azure C with low purity are added, the mixture is cooled down to 10°C by an ice-water bath, and Fmoc-Cl is added in lots to react in the ice-water bath and then react at room temperature to produce a viscous product; the supernatant is poured off, the viscous product is dissolved using dichloromethane, insoluble impurities are filtered out, and the filtrate is washed with distilled water; drying over anhydrous sodium sulfate and concentrating to obtain an intermediate (Fmoc)₂-Azure C; and (Fmoc)₂-Azure C is dissolved in DMF, diethylamine is dropwise added slowly in an ice-water bath, reacting in the ice-water bath, ether is added to separate out a black solid, and vacuum filtration, washing with ether, and vacuum drying at room temperature are carried out to obtain Azure C with high purity.

Further, Fmoc-Cl is added in lots to react for 1 hour in the ice-water bath at first and then react for 2 hours at room temperature to produce a viscous product.

Further, (Fmoc)₂-Azure C is dissolved in DMF, and diethylamine is dropwise added slowly in the ice-water bath to react for 1 hour in the ice-water bath.

One of the objects of the present invention is to provide a method for purification of Azure C using di-tert-butyl dicarbonate as an amino protection reagent, and the method comprises the following steps: weighing Azure C with low purity and dissolving the same in DMF, adding triethylamine and Boc₂O for reaction to obtain a viscous product, and dissolving the viscous product in an organic solvent and purifying the product to obtain an intermediate (Boc)2-Azure C; and dissolving the intermediate (Boc)2-Azure C in an organic solvent, adding trifluoroacetic acid to react at room temperature, carrying out concentration to evaporate the solvent, adding acetonitrile for dissolving, adding a hydrogen chloride ether solution to separate out a black solid, and carrying out vacuum filtration, washing and drying to obtain Azure C with high purity.

Further, triethylamine and Boc₂O are added in an ice-water bath.

Further, the organic solvent is dichloromethane.

Further, the viscous product dissolved in dichloromethane is dried and concentrated, and purified by column chromatography to obtain an intermediate product intermediate (Boc)₂-Azure C.

Further, the eluent for the column chromatography is ethyl acetate/petroleum ether=1/10.

Further, Azure C with low purity is weighed and dissolved in DMF, triethylamine and Boc₂O are added in an ice-water bath to react in the ice-water bath and then react at room temperature, suction filtration is carried out, the filtrate is poured into ice water and stirred to separate out a viscous product, the supernatant is poured off, and the viscous product is dissolved in dichloromethane, dried by anhydrous sodium sulfate, concentrated, and is purified by column chromatography to obtain an intermediate (Boc)₂-Azure C; and (Boc)₂-Azure C is dissolved in dichloromethane, trifluoroacetic acid is added to react at room temperature, concentration is carried out to evaporate the solvent, acetonitrile is added and stirred for dissolving, a hydrogen chloride ether solution is dropwise added to separate out a black solid, and vacuum filtration, washing with acetonitrile, and vacuum drying at room temperature are carried out to obtain Azure C with high purity.

One of the objects of the present invention is to provide a method for purification of Azure C using triphenylchloromethane as an amino protection reagent, and the method comprises the following steps: weighing Azure C with low purity and Trt-Cl and dissolving them in DMF, adding triethylamine to react at room temperature, filtering out insoluble impurities, diluting the filtrate by dichloromethane, washing the filtrate with distilled water, and carrying out drying and concentration, and purification using column chromatography to obtain an intermediate Trt-Azure C; and dissolving the intermediate Trt-Azure C in an organic solvent, adding trifluoroacetic acid to react at room temperature, carrying out concentration to evaporate the solvent, adding acetonitrile for dissolving, adding a hydrogen chloride ether solution to separate out a black solid, and carrying out vacuum filtration, washing and drying to obtain Azure C with high purity.

Further, the intermediate Trt Azure C is dissolved in an organic solvent dichloromethane.

Further, trifluoroacetic acid is added to react at room temperature.

Further, the purification is carried out by column chromatography.

Further, the eluent for column chromatography is ethyl acetate/methanol=20/1. Further, Azure C with low purity and Trt-Cl are weighed and dissolved in DMF, triethylamine is dropwise added to react at room temperature, insoluble impurities are filtered out, the filtrate is diluted by dichloromethane and washed with distilled water, dried over anhydrous sodium sulfate and evaprorated, and purified by column chromatography to obtain an intermediate Trt-Azure C; and Trt-Azure C is dissolved in dichloromethane, trifluoroacetic acid is added to react at room temperature, and the solvent is evaporated, acetonitrile is added and stirred for dissolving, a hydrogen chloride ether solution is dropwise added to separate out a black solid, and vacuum filtration, washing with acetonitrile, and vacuum drying at room temperature are carried out to obtain Azure C with high purity.

The beneficial effects of the present invention are as follows: in the present invention, an amino protection reagent is used to form amino-protected Azure C (i.e., an intermediate in the purification process) that is prone to dissolving in various organic solvents from commercially available Azure C (i.e., low purity), then the intermediate is purified, and after deprotection of the intermediate, Azure C with high purity is finally obtained. The reagent and method for purification described in the present invention are used to achieve a very large treatment capacity in a single purification, effectively improve the purification efficiency of commercially available Azure C and reduce the preparation and purification costs. Moreover, the dosage of Azure C on a biosensor is more precise, and the using effect is improved. The method for purification described in the present invention achieves a very large treatment capacity in one time, and obtains a high yield of products with high purity, which can effectively meet the preparation requirements of biosensors for blood glucose test and the like and improve the production efficiency.

### Brief Description of the Drawings

FIG. 1 is the LC-MS spectrum of commercially available Azure C (Azure C with low purity before purification).
FIG. 2 is the LC-MS spectrum of commercially available Azure C, which is a mass spectrum corresponding to the peak position at T=1.55 min in the mass spectrometry scan.
FIG. 3 is the LC-MS spectrum of Azure C purified using Fmoc as a protecting group in Example 1.
FIG. 4 is the LC-MS spectrum of Azure C purified using Boc as a protecting group in Example 2.
FIG. 5 is the LC-MS spectrum of Azure C purified using Trt as a protecting group in Example 3.

### Detailed Description of the Embodiments

The present invention will be described below in detail in conjunction with specific embodiments. These specific embodiments are only limited enumeration without departing from the spirit of the present invention, and do not exclude other specific embodiments derived from the combination of the prior art and the present invention by a person of ordinary skill in the art.

The method for purification of commercially available Azure C in the present invention includes:
Step 1: the amino protection reagent reacts with Azure C with low purity to obtain amino-protected Azure C.
Step 2: amino-protected Azure C obtained in Step 1 is prone to dissolving in various organic solvents, and amino-protected Azure C with high purity (intermediate) is obtained through conventional separation methods (for example, column chromatography).
Step 3, the intermediate obtained in Step 2 is subjected to amino deprotection to obtain Azure C with high purity.

The common amino protection reagent is selected from, but not limited to, fluorenylmethyloxycarbonyl chloride (Fmoc-Cl), di-tert-butyl dicarbonate (Boc2O), riphenylchloromethane (Trt-Cl), carbobenzoxy chloride (Cbz-Cl), paratoluensulfonyl chloride (Tos-Cl), trifluoroacetic anhydride (TFAA).

The LC-MS (LC-MS instrument) analysis method of Azure C is as follows: Instrument: Waters Acquity UPLC H-Class+SQD2 LC-MS instrument, chromatographic column: Acquity UPLC^{®} BEH C18, 1.7µm, 21×50mm Column. Flow phase: acetonitrile/water (0.1% formic acid), acetonitrile% = 10-90% (3min). Flow rate: 0.4ml/min.

Test wavelength: 200-800nm full scan.

Column temperature: 40°C.

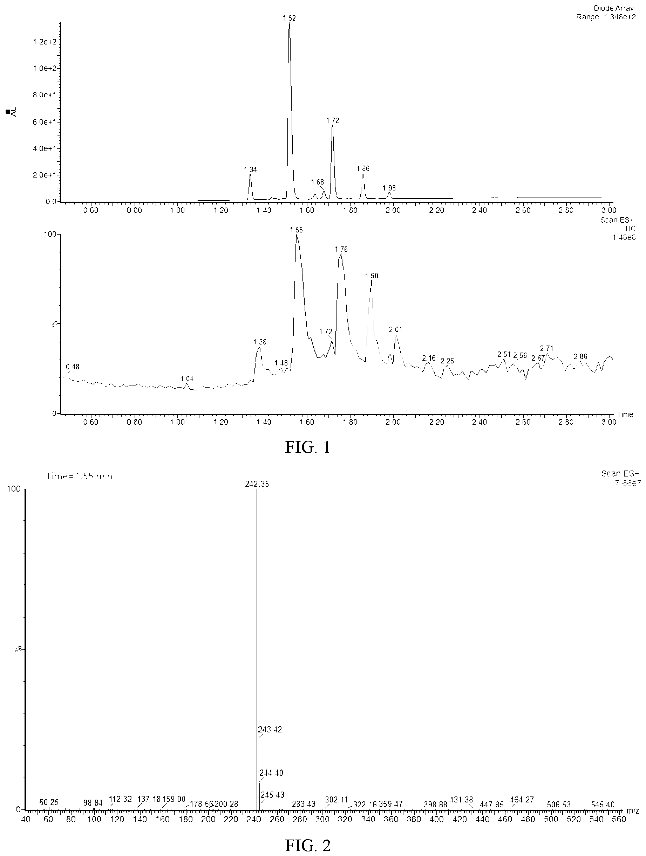

The content of commercially available Azure C is 40% via the above LC-MS analysis, as shown in FIG. 1. [M-Cl]⁺=242.35 via analysis of the mass spectrum of Azure C, as shown in FIG. 2.

### Example 1 Purification of Azure C using Fmoc-Cl as amino protection reagent

A method for purification of Azure C using fluorenylmethyloxycarbonyl chloride (Fmoc-Cl) as an amino protection reagent. 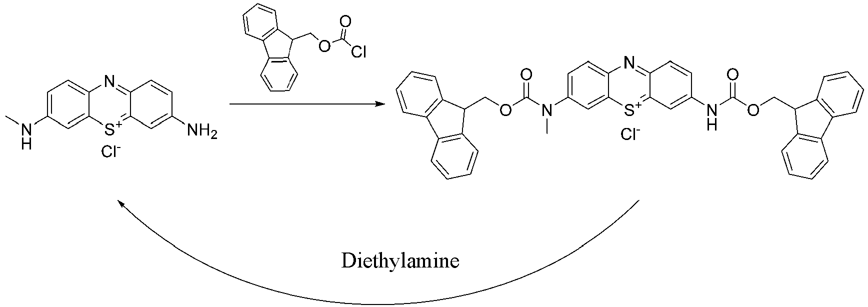 15g (141.5mol) of sodium carbonate was weighed, 150ml of water was added and stirred to dissolve sodium carbonate, then 150ml of dioxane and 10g (36mmol) of commercially available Azure C with a content of 40% were added, and then the mixture was cooled down to 10°C by an ice-water bath. 23.28g (90mmol) of Fmoc-Cl was added in lots to react for 1 hour in the ice-water bath at first and then react for 2 hours at room temperature to produce a viscous product. The supernatant was poured off, the viscous product was dissolved using dichloromethane, insoluble impurities were filtered out, and the filtrate was washed with distilled water. Dried over anhydrous sodium sulfate and evaprorated to obtain 3.4g of an intermediate (Fmoc)₂-Azure C. (Fmoc)₂-Azure C was amino-protected Azure C.

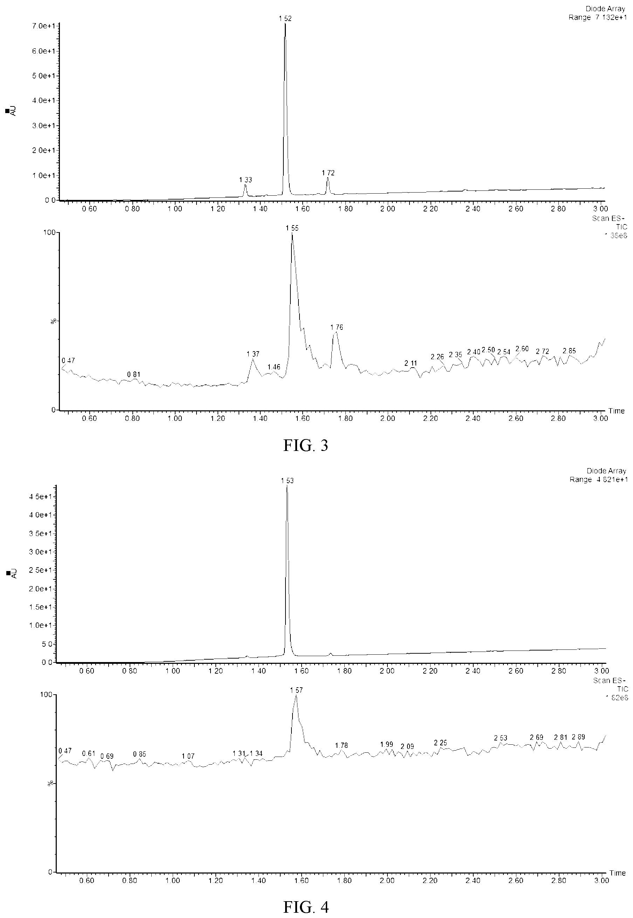

The prepared intermediate (Fmoc)₂-Azure C was dissolved in 75ml of DMF, and 20ml of diethylamine was dropwise added slowly in the ice-water bath to react for 1 hour in the ice-water bath. 500ml of ether was added to separate out a black solid, and vacuum filtration, washed with ether, and vacuum dried at room temperature were carried out to obtain 2.3g of Azure C (purity: 84.6%), as shown in FIG. 3.

### Example 2 Purification of Azure C using Boc₂O as amino protection reagent

A method for purification of Azure C using di-tert-butyl dicarbonate (Boc2O) as an amino protection reagent. 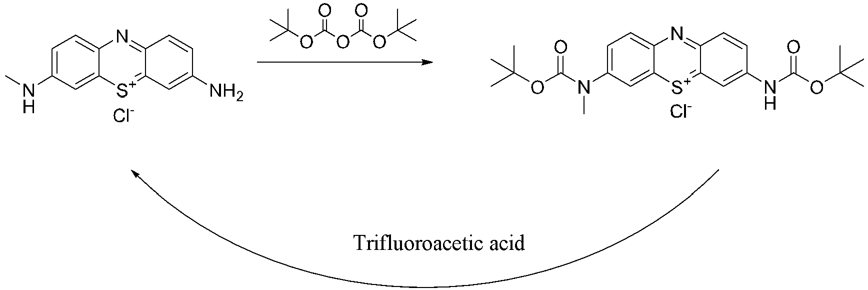 5g (18mmol) of 40% commercially available Azure C was weighed, and dissolved in 100ml of DMF, and 15m1 (108mmol) of triethylamine and 16.5ml (72mmol) of Boc₂O were added in an ice-water bath to react for 1 hour in the ice-water bath and then react for 3 hours at room temperature. Vacuum filtration was carried out, the filtrate was poured into ice water and stirred to separate out a viscous product, the supernatant was poured off, and the viscous product was dissolved in dichloromethane, and dried over anhydrous sodium sulfate and evaprorated. The residue was chromatographed on chromatography Column (for example, selecting a SiO2 silica gel column) with ethyl acetate/ methyl alcohol=20/1 to obtain 1.6g of an intermediate (Boc)₂-Azure C. (Boc)₂-Azure C was amino-protected Azure C.

The prepared intermediate (Boc)₂-Azure C was dissolved in 15ml of dichloromethane, and 3ml of trifluoroacetic acid was added to react for 4 hours at room temperature. The solvent was evaporated, 30ml of acetonitrile was added and stirred for dissolving, and 3ml of a hydrogen chloride ether solution was dropwise added to separate out a black solid. Vacuum filtration, washed with acetonitrile, and vacuum dried at room temperature were carried out to obtain 1g of Azure C (purity: 95%), as shown in FIG. 4.

### Example 3 Purification of Azure C using Trt-Cl as amino protection reagent

A method for purification of Azure C using Trt-Cl as an amino protection reagent. 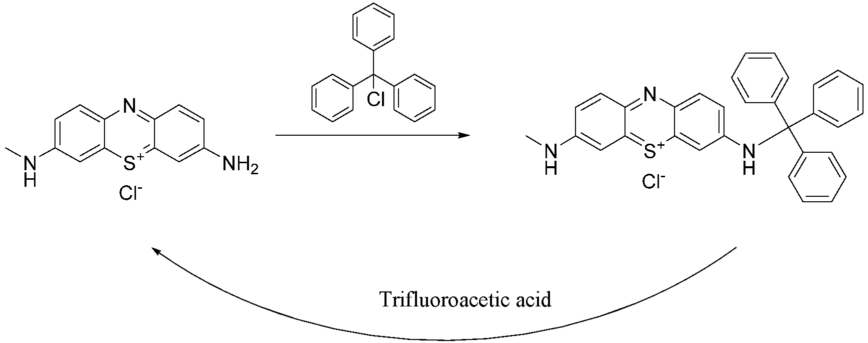 5g (18mmol) of 40% Azure C and 15g (54mmol) of Trt-Cl were weighed, and dissolved in 100ml of DMF, and then 12.5ml (90mmol) of triethylamine was dropwise added to react for 1 hour at room temperature. Vacuum filtration was carried out, the filtrate was diluted with 400ml of dichloromethane, and washed with distilled water, and dried over anhydrous sodium sulfate and evaprorated. The residue was chromatographed on chromatography Column (for example, selecting a SiO2 silica gel column) with ethyl acetate/ methyl alcohol=20/1 to obtain 2.2g of an intermediate Trt-Azure C. Trt-Azure C was amino-protected Azure C.

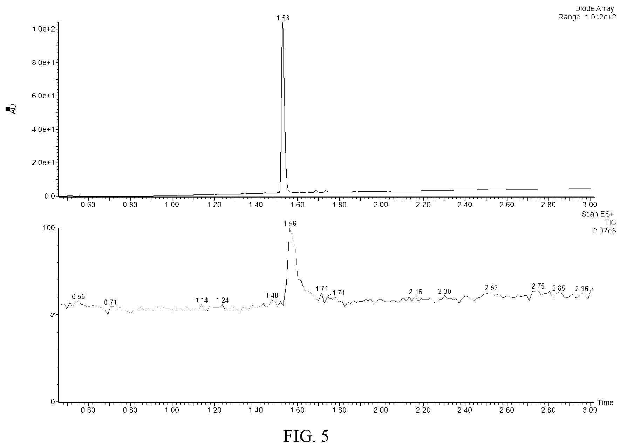

The prepared intermediate Trt-Azure C was dissolved in 30ml of dichloromethane, and 3ml of trifluoroacetic acid was added to react for 1 hour at room temperature. The solvent was evaporated, 30ml of acetonitrile was added and stirred for dissolving, and 3ml of a hydrogen chloride ether solution was dropwise added to separate out a black solid. Vacuum filtration, washed with acetonitrile, and vacuum dried at room temperature were carried out to obtain 1.24g of Azure C (purity: 97%), as shown in FIG. 5.

### Example 4 Direct preparation and purification of commercially available Azure C using HPLC (Comparative example)

As a comparative example of the present invention, a method for direct purification of commercially available Azure C using HPLC was as follows:
Instrument: Waters Prepl50 preparation liquid phase chromatogram Instrument, chromatographic column: SunFire^{®}Prep C18 OBD^{™} 5µm 19×150mm Column.
Flow phase: acetonitrile/water (0.05% trifluoroacetic acid), acetonitrile%= 15-55%(10min).
Flow rate: 20ml/min.
Test wavelength: 610nm.
Column temperature: room temperature
Sample concentration: 5mg/ml in N,N-dimethylformamide, sample injection volume: 1ml.

The primary product (Azure C) at T = 3.6-4.4min was collected.

After analysis of Azure C directly purified using HPLC, 1-2mg of Azure C could be collected each time, and the purity of Azure C obtained from purification was ≥ 99%. Through analysis and comparison of experimental results of Examples 1, 2, 3 and Comparative example (Example 4), it can be seen that by using the method for purification in the present invention, the treatment capacity is very large in a single purification, the amount that can be treated is at least at the level of "gram", and the amount of Azure C with high purity obtained from purification is also very large, which is at least at the level of "gram". However, by using HPLC to directly purify Azure C (Example 4), the treatment capacity is very small in a single purification, the amount that can be treated is only at the level of "milligram", and the amount of Azure C with high purity obtained from purification is also very small, which is only at the level of "milligram". Therefore, the present invention can effectively increase the treatment capacity of a single purification and improve the purification yield, with high purification efficiency and low cost. Through experimental verification, Azure C purified by the method of the present invention serves as an electron mediator reagent for blood glucose test to prepare blood glucose biosensors. The prepared biosensors can meet various test requirements such as blood glucose test. Therefore, the Azure C purified using the method in the present invention can be used as the electron mediator of biosensors such as a blood glucose biosensor.

## Claims

1. A method for purification of an electron mediator, the electron mediator having an amino, wherein the method comprises the following steps: weighing an electron mediator with low purity; reacting an amino protection reagent with the electron mediator to obtain an amino-protected electron mediator, dissolving the amino-protected electron mediator in an organic solvent, and carrying out extraction to obtain an amino-protected electron mediator with high purity; and reacting the amino-protected electron mediator with high purity with a deprotection reagent for amino deprotection, thus obtaining an electron mediator with high purity.

2. The method for purification according to claim 1, wherein the electron mediator is selected from Azure C.

3. The method for purification according to claim 1, wherein the amino protection reagent is selected from fluorenylmethyloxycarbonyl chloride, di-tert-butyl dicarbonate, triphenylchloromethane, carbobenzoxy chloride, paratoluensulfonyl chloride or trifluoroacetic anhydride.

4. The method for purification according to one of claims 2 to 3, wherein the structural formula of the amino-protected electron mediator 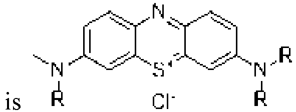 wherein R = H or X, X = amino protecting group, and the number of the amino protecting groups X varies from 1 to 3.

5. The method for purification according to claim 4, wherein the amino protecting group is selected from Fmoc, Boc, Trt, Cbz, Tos or TFA.

6. The method for purification according to claim 1, wherein the deprotection reagent is selected from diethylamine, piperidine, trifluoroacetic acid, hydrogen bromide/acetic acid, or sodium hydroxide.

7. The method for purification according to claim 6, wherein the deprotection reagent diethylamine or piperidine is used for deprotection of Fmoc, trifluoroacetic acid is used for deprotection of Boc or Trt, hydrogen bromide/acetic acid is used for deprotection of Cbz or Tos, and sodium hydroxide is used for deprotection of TFA.

8. The method for purification according to claim 1, wherein the method for extracting an amino-protected electron mediator with high purity is column chromatography.

9. The method for purification according to claim 1, wherein the amino protection reagent is Fmoc-Cl, and the method comprises the following steps: preparing a mixed solution of Azure C with low purity, sodium carbonate and dioxane; adding Fmoc-Cl to the mixed solution for reaction to obtain a viscous product; dissolving the viscous product in an organic solvent, filtering out insoluble impurities, washing the filtrate with distilled water, and drying over anhydrous sodium sulfate and evaprorate to obtain an intermediate (Fmoc)₂-Azure C; and dissolving the intermediate (Fmoc)₂-Azure C in DMF, adding diethylamine for reaction, then adding ether to separate out a black solid, and carrying out vacuum filtration, washing and drying to obtain Azure C with high purity.

10. The method for purification according to claim 9, comprising the following steps: weighing sodium carbonate and stirring in water to dissolve sodium carbonate, then adding dioxane and Azure C with low purity, cooling down the mixture to 10°C by an ice-water bath, and adding Fmoc-Cl in lots to react in the ice-water bath and then react at room temperature to produce a viscous product; pouring off the supernatant, dissolving the viscous product by dichloromethane, filtering out insoluble impurities, and washing the filtrate with distilled water; drying over anhydrous sodium sulfate and evaprorate to obtain an intermediate (Fmoc)2-Azure C; and dissolving (Fmoc)2-Azure C in DMF, dropwise adding diethylamine slowly in an ice-water bath to react in the ice-water bath, adding ether to separate out a black solid, and carrying out vacuum filtration, washing with ether, and vacuum drying at room temperature to obtain Azure C with high purity.

11. The method for purification according to claim 10, comprising the following steps: weighing 15g (141.5mol) of sodium carbonate, adding 150ml of water and stirring to dissolve sodium carbonate, then adding 150ml of dioxane and 10g of Azure C with low purity, and cooling down the mixture to 10°C by an ice-water bath; adding 23.28g (90mmol) of Fmoc-Cl in lots to react for 1 hour in the ice-water bath and then react for 2 hours at room temperature to produce a viscous product; pouring off the supernatant, dissolving the viscous product using dichloromethane, filtering out insoluble impurities, and washing the filtrate with distilled water; drying over anhydrous sodium sulfate and evaprorate to obtain an intermediate (Fmoc)₂-Azure C; and dissolving the prepared (Fmoc)₂-Azure C in 75ml of DMF, dropwise adding 20ml of diethylamine slowly in the ice-water bath to react for 1 hour in the ice-water bath, adding 500ml of ether to separate out a black solid, and carrying vacuum filtration, washing with ether, and vacuum drying at room temperature to obtain Azure C with high purity.

12. The method for purification according to claim 1, wherein the amino protection reagent is Boc₂O, and the method comprises the following steps: weighing Azure C with low purity and dissolving the same in DMF, adding triethylamine and Boc₂O for reaction to obtain a viscous product, and dissolving the viscous product in an organic solvent and purifying the product to obtain an intermediate (Boc)₂-Azure C; and dissolving the intermediate (Boc)₂-Azure C in an organic solvent, adding trifluoroacetic acid to react at room temperature, carrying out concentration to evaporate the solvent, adding acetonitrile for dissolving, adding a hydrogen chloride ether solution to separate out a black solid, and carrying out vacuum filtration, washing and drying to obtain Azure C with high purity.

13. The method for purification according to claim 12, comprising the following steps: weighing Azure C with low purity and dissolving the same in DMF, adding triethylamine and Boc₂O in an ice-water bath to react in the ice-water bath and then react at room temperature, carrying out suction filtration, pouring the filtrate into ice water and stirring to separate out a viscous product, pouring off the supernatant, dissolving the viscous product in dichloromethane, drying over anhydrous sodium sulfate and evaprorate, and carrying out purification using column chromatography to obtain an intermediate (Boc)₂-Azure C; and dissolving (Boc)₂-Azure C in dichloromethane, adding trifluoroacetic acid to react at room temperature, carrying out concentration to evaporate the solvent, adding acetonitrile and stirring for dissolving, dropwise adding a hydrogen chloride ether solution to separate out a black solid, and carrying out vacuum filtration, washing with acetonitrile, and vacuum drying at room temperature to obtain Azure C with high purity.

14. The method for purification according to claim 13, comprising the following steps: weighing 5g of Azure C with low purity and dissolving the same in 100ml of DMF, and adding 15ml (108mol) of triethylamine and 16.5ml (72mmol) of Boc₂O in an ice-water bath to react for 1 hour in the ice-water bath and then react for 3 hours at room temperature; carrying out vacuum filtration, pouring the filtrate into ice water and stirring to separate out a viscous product, pouring off the supernatant, dissolving the viscous product in dichloromethane, and drying over anhydrous sodium sulfate and evaprorate; the residue was chromatographed on chromatography Column with ethyl acetate/ methyl alcohol=20/1,with the chromatographic column selected from a SiO₂ silica gel column; dissolving the prepared intermediate (Boc)₂-Azure C in 15ml of dichloromethane, and adding 3ml of trifluoroacetic acid to react for 4 hours at room temperature; carrying out concentration to evaporate the solvent, adding 30ml of acetonitrile and stirring for dissolving, and dropwise adding 3ml of a hydrogen chloride ether solution to separate out a black solid; and carrying out vacuum filtration, washing with acetonitrile, and vacuum drying at room temperature to obtain Azure C with high purity.

15. The method for purification according to claim 1, wherein the amino protection reagent is Trt-Cl, and the method comprises the following steps: weighing Azure C with low purity and Trt-Cl and dissolving them in DMF, adding triethylamine to react at room temperature, filtering out insoluble impurities, diluting the filtrate by dichloromethane, washing the filtrate with distilled water, and drying over anhydrous sodium sulfate and evaprorate, and purification using column chromatography to obtain an intermediate Trt-Azure C; and dissolving the intermediate Trt-Azure C in an organic solvent, adding trifluoroacetic acid to react at room temperature, carrying out concentration to evaporate the solvent, adding acetonitrile for dissolving, adding a hydrogen chloride ether solution to separate out a black solid, and carrying out vacuum filtration, washing and drying to obtain Azure C with high purity.

16. The method for purification according to claim 1, comprising the following steps: weighing Azure C with low purity and Trt-Cl and dissolving them in DMF, then dropwise adding triethylamine to react at room temperature, filtering out insoluble impurities, diluting the filtrate by dichloromethane, washing the filtrate with distilled water, and drying over anhydrous sodium sulfate and evaprorate, and purification using column chromatography to obtain an intermediate Trt-Azure C; and dissolving the Trt-Azure C in dichloromethane, adding trifluoroacetic acid to react at room temperature, carrying out concentration to evaporate the solvent, adding acetonitrile and stirring for dissolving, dropwise adding a hydrogen chloride ether solution to separate out a black solid, and carrying out vacuum filtration, washing and drying to obtain Azure C with high purity.
